# EUROPÄISCHE PATENTSCHRIFT

(11) **EP 3 691 867 B1**
(45) Veröffentlichungstag und Bekanntmachung des Hinweises auf die Patenterteilung: **21.12.2022**
(21) Anmeldenummer: 18779662.8
(22) Anmeldetag: 28.09.2018
(51) Int. Cl.: B29C 64/364, B29C 64/255, B29C 64/295, B29C 64/25, B33Y 30/00, B33Y 40/00, A61L 2/00, B22F 3/105, B29C 64/10

(54) **FLUIDVERSORGUNGSSYSTEM FÜR EINEN 3D-DRUCKER**
FLUID SUPPLY SYSTEM FOR A 3D PRINTER
SYSTÈME D'ALIMENTATION EN FLUIDE D'UNE IMPRIMANTE 3D

(30) Priorität: 02.10.2017 DE 102017122849
(43) Veröffentlichungstag der Anmeldung: 12.08.2020
(73) Patentinhaber: Kumovis GmbH, 81369 München (DE)
(72) Erfinder: LEONHARDT, Stefan, 85748 Garching (DE); FISCHER, Stefan, 85748 Garching (DE); PAMMER, Sebastian, 85748 Garching (DE); HAERST, Miriam, 85748 Garching (DE)
(74) Vertreter: DTS Patent- und Rechtsanwälte Schnekenbühl und Partner mbB
(86) Internationale Anmeldenummer: PCT/EP2018/076466
(87) Internationale Veröffentlichungsnummer: WO 2019/068581

(56) Entgegenhaltungen:
- EP-A1- 2 492 084
- DE-A1-102006 014 835
- US-A1- 2017 128 601

## Beschreibung

Die vorliegende Offenbarung betrifft ein Fluidversorgungssystem für wenigstens einen 3D-Drucker mit wenigstens einer Fluiddruckerzeugungsvorrichtung zur Erzeugung eines druckbeaufschlagten Fluidstroms und mit wenigstens einer Fluiderhitzungsvorrichtung zur Erhitzung des Fluidstroms.

Im Zusammenhang mit dem 3D-Druck von Kunststoffen insbesondere für medizinische Anwendungen (z.B. für Implantate) steht vor allem die derzeit erzielbare Bauteilqualität im Fokus vieler wissenschaftlicher Untersuchungen. Zwei der wichtigsten Herausforderungen, die im Hinblick auf Bauteilqualität eine entscheidende Rolle spielen sind die Bauteiltoleranz und die Bauteilsterilität bzw. der Partikelniederschlag.

So ist aus der DE 10 2015 111 504 A1 bereits eine 3D-Druckvorrichtung, insbesondere eine FFF-Druckvorrichtung, mit zumindest einer Druckkopfeinheit bekannt, wobei die Druckkopfeinheit in zumindest einem Betriebszustand dazu vorgesehen ist, ein zumindest teilweise von einem Hochleistungskunststoff, insbesondere einem Hochleistungsthermoplast, gebildetes Druckmaterial aufzuschmelzen.

Ferner offenbart die EP 2 261 009 A1 eine Vorrichtung und ein Verfahren zum Herstellen eines dreidimensionalen Objekts, wobei die Vorrichtung eine Vakuumpumpe aufweist, die an ein Zuführreservoir gekoppelt ist, um einen Luftstrom durch das Zuführreservoir zu erzeugen.

Zudem ist in der EP 3 023 228 A1 eine additive Fertigungsvorrichtung gezeigt, die ein Gasströmungssystem aufweist, um einen Gasstrom über dem Bereich der Bauplattform der additiven Fertigungsvorrichtung bereitzustellen.

Im Übrigen ist in der EP 3 173 233 A1 eine dreidimensionale Fertigungsvorrichtung offenbart, die ein Verarbeitungsraum aufweist, der von einer dafür vorgesehenen Verarbeitungsraum-Heizeinheit beheizt wird.

Außerdem offenbart die US 6,033,301 A eine kombinierte Lüfter-Filter-Einheit, die zur Filterung der Luft eines Luftkreislaufs in einem Reinraum vorgesehen ist.

Weiterhin ist in der US 6,722,872 B1 eine dreidimensionale Modellierungsvorrichtung gezeigt, die dazu vorgesehen ist dreidimensionale Objekte innerhalb einer beheizten Baukammer aufzubauen.

Darüber hinaus ist in der US 6,817,941 B1 ein Diffusor zur Erzeugung eines gleichmäßigen Luftstroms innerhalb einer Prozesskammer gezeigt, die z.B. bei der Fertigung von Halbleiterchips Anwendung findet.

Des Weiteren zeigt die US 2015/110911 A1 eine Umgebungs-Überwachungs- bzw. Steuerungseinheit, die beispielsweise als eine Schnittstelle bei additiven Fertigungstechnologien zu ihren jeweiligen Umgebungen verwendet wird.

Im Übrigen ist in der WO 2016/063198 A1 ein Verfahren und eine Vorrichtung zur Herstellung von dreidimensionalen Objekten durch "Fused Deposition Modelling" gezeigt, wobei die Herstellungsvorrichtung Strahlungsheizelemente aufweist, welche eine ihnen ausgesetzte Oberfläche des herzustellenden Objekts erhitzen können.

Zudem ist aus der WO 2017/040675 A1 eine Reinraumtechnologie für 3D Drucker und sogenannte Bioprinter bekannt.

Aus der WO 2017/108477 A1 kann weiter ein Verfahren zur Herstellung eines dreidimensionalen Gegenstandes mit einem "Fused Deposition Modelling"-Drucker entnommen werden.

Aus der US 2017/012860 A1 ist eine Vorrichtung zum sterilen 3D-Druck bekannt.

Ausgehend von den im Stand der Technik vorgeschlagenen Lösungen besteht bei diesen additiven Fertigungsvorrichtungen weiterhin die Problematik eines hohen Bauteilverzugs sowie einer für medizinische Anwendungen nicht ausreichenden Sterilität sowie Freiheit von Keimen und Partikeln. Eine minimale Kontamination des Bauteils ist anzustreben und wird derzeit noch nicht erreicht.

Es ist daher die Aufgabe der vorliegenden Erfindung, ein Fluidversorgungssystem für einen 3D-Drucker der eingangs genannten Art in vorteilhafter Weise weiterzubilden, insbesondere dahingehend, dass der Verzug und die Sterilität eines mittels des 3D-Druckers hergestellten Bauteils verbessert wird und die Energieeffizienz des Fluidversorgungssystems verbessert wird.

Diese Aufgabe wird erfindungsgemäß gelöst durch einen 3D-Drucker mit den Merkmalen des Anspruchs 1. Danach ist vorgesehen, dass ein 3D-Drucker, insbesondere ein FFF-3D-Drucker, mit einem Fluidversorgungssystem, das wenigstens einer Fluiddruckerzeugungsvorrichtung zur Erzeugung eines Fluidstroms und wenigstens einer Fluiderhitzungsvorrichtung zur Erhitzung des Fluidstroms versehen ist, wobei der 3D-Drucker wenigstens eine Baukammer aufweist, die mittels wenigstens eines Baukammer-Gehäuses gegenüber der Umgebung des 3D-Druckers begrenzt und fluiddicht abgeschlossen ist, wobei die Fluiddruckerzeugungsvorrichtung, die Fluiderhitzungsvorrichtung und das Baukammer-Gehäuse in Fluidverbindung stehen, wodurch die Baukammer mittels des Fluidstroms durchströmbar ist, und wobei mittels der Fluiddruckerzeugungsvorrichtung, der Fluiderhitzungsvorrichtung und des Baukammer-Gehäuses ein geschlossener Fluidkreislauf für den Fluidstrom ausgebildet ist, der vor Eintritt in die Baukammer mittels der Fluiderhitzungsvorrichtung erhitzt ist, wobei das Fluidversorgungssystem im betriebsbereiten Zustand so ausgestaltet ist, dass die Baukammer mittels des Fluidstroms in Form einer im Wesentlichen laminaren Strömung durchströmbar ist, und wobei das Fluidversorgungssystem wenigstens einen Baukammereintrittsbereich aufweist, der stromaufwärts des Baukammer-Gehäuses angeordnet ist und in dem wenigstens eine Strömungsausrichtungseinheit angeordnet ist, wobei der Baukammereintrittsbereich in Form eines Diffusors ausgebildet ist.

Vorteilhaft ist es möglich, ein Ventil oder einen Zufluss von, vorteilhafterweise gefilterter, Umgebungsluft vorzusehen, um den Bauraum zu temperieren. Grundsätzlich kann vorgesehen sein, dass auch Luftspalte oder Luftzuflüsse von externer Luft vorgesehen sein können bzw. tolerierbar sind. Eine komplette Fluiddichtigkeit ist nicht notwendig. Zudem kann zusätzlich zugeführte kalte Luft mit dem beheizten Kreislauf gemischt werden, um den Bauraum auch nach dem Druckaufbau zu kühlen.

Die Erfindung basiert auf dem Grundgedanken, dass aufgrund des Aufbaus eines geschlossenen Fluidkreislaufs das die Baukammer des 3D-Druckers bereits durchströmte und vor dessen Eintritt erhitzte Fluid nach Austritt nicht ungenutzt aus der Baukammer nicht in die Umgebung des 3D-Druckers abgegeben wird. Vielmehr kann das noch teilweise erhitzte Fluid, das die Baukammer verlässt, aufgrund des geschlossenen Fluidkreislaufs erneut der Baukammer zugeführt werden. Diese Art des Fluidkreislaufs ist insbesondere deshalb so vorteilhaft, weil das die Baukammer verlassende Fluid noch immer eine gegenüber ihrer Umgebungstemperatur deutlich erhöhte Temperatur aufweist. Demzufolge kann die aufzubringende Energiemenge für die erneute Erhitzung des Fluids deutlich reduziert werden. Somit kann einerseits der Energiebedarf zur Fluiderhitzung deutlich gesenkt werden. Andererseits kann die Fluiderhitzungsvorrichtung im Vergleich zum Stand der Technik deutlich platzsparender bzw. kompakter dimensioniert werden, da sie das Fluid nicht ausgehend von der Umgebungstemperatur auf die gewünschte BaukammerTemperatur erhitzen muss. Die Erhitzung der Baukammer ist insbesondere für eine verzugsarme Verarbeitung von teilkristallinen Thermoplasten optimal. Durch die zumindest teilweise Erhitzung der Baukammer über einen stromaufwärts erhitzten Fluidstrom kann eine besonders homogene Temperaturverteilung innerhalb der Baukammer realisiert werden. Diese homogene Temperaturverteilung wirkt sich zusätzlich positiv auf eine Verzugsreduzierung bei der Verarbeitung von Thermoplasten aus. Insgesamt kann das Fluidversorgungssystem demzufolge deutlich wirtschaftlicher bzw. kostengünstiger ausgelegt werden. Darüber hinaus ist es nicht mehr erforderlich, die Partikel aus der Umgebungsluft herausfiltern zu müssen, da stets eine Umwälzung des bereits gefilterten Luftstroms erfolgt und dieser Luftstrom erneut gefiltert wird. Dadurch sind insgesamt weniger Partikel aus dem Luftstrom herauszufiltern, weshalb auch die Filter eine längere Lebensdauer erreichen können.

Erfindungsgemäss ist vorgesehen, dass das Fluidversorgungssystem im betriebsbereiten Zustand so ausgestaltet ist, dass die Baukammer mittels des Fluidstroms in Form einer im Wesentlichen laminaren Strömung durchströmbar ist. Die laminare Durchströmung der Baukammer (mit einem Laminar-Air-Flow insbesondere zur Erzeugung von Reinraumbedingungen) hat insbesondere aufgrund ihrer gerichteten Strömungscharakteristik (ohne Querströmungen) den Vorteil, dass die Baukammer möglichst wenig mit Partikeln oder Keimen kontaminiert wird, welche in dem Fluid enthalten sind oder durch die Strömung mitgerissen werden. Da die vorliegende Erfindung insbesondere auch im Bereich der Medizintechnik (z.B. zur Herstellung von Implantaten) verwendet wird, wirkt sich eine möglichst geringe Kontamination des herzustellenden Bauteils besonders positiv auf dessen Sterilität aus. In diesem Zusammenhang ist es ferner denkbar, dass die Baukammer mittels des Fluidstroms in Form einer im Wesentlichen vertikalen laminaren Strömung durchströmbar ist.

Weiter ist vorstellbar, dass der 3D-Drucker wenigstens einen innerhalb der Baukammer mehrachsig und/oder mehrdimensional, insbesondere dreidimensional, bewegbaren Druckkopf und wenigstens eine Bauplattform aufweist, die von der laminaren Strömung des Fluidstroms umgeben sind. Durch den dreidimensional bewegbaren Druckkopf und die Umströmung von Druckkopf und Bauplattform mit einer laminaren Strömung des Fluidstroms kann im Laminar-Air-Flow das zu fertigende Produkt gedruckt werden, was vorteilhaft für die Sterilität und Keimfreiheit sowie Freiheit von Partikeln ist. Die laminare Umströmung dieser Bauteile senkt insbesondere deren Verschmutzungen bzw. Ablagerungen durch in der Luft vorhandenen Partikel oder Keime. Da diese beiden Baugruppen unmittelbar mit dem herzustellenden Bauteil in Kontakt stehen, sind deren keimfreien, partikelfreien und schmutzfreien Oberflächen unerlässlich.

Zudem weist das Fluidversorgungssystem wenigstens einen Baukammereintrittsbereich auf, der stromaufwärts des Baukammer-Gehäuses angeordnet ist und in dem wenigstens eine Strömungsausrichtungseinheit angeordnet ist. Die Strömungsausrichtungseinheit dient dazu, den Fluidstrom vor Eintritt in die Baukammer zu beruhigen. Da der Fluidstrom stromaufwärts der Strömungsausrichtungseinheit durch die Fluiddruckerzeugungsvorrichtung, Fluiderhitzungsvorrichtung sowie Rohrleitungen und Krümmer verwirbelt ist, ist eine Ausrichtung des Fluidstroms vor Eintritt in die Baukammer daher unumgänglich. Der Baukammereintrittsbereich ist in Form eines Diffusors ausgebildet. Als eine mögliche geometrische Ausgestaltung des Diffusors kann ein dünnwandiger Hohlkegel dienen, dessen Strömungsquerschnittsfläche sich stromaufwärts in Richtung der Baukammer linear erweitert.

Außerdem ist es möglich, dass die Strömungsausrichtungseinheit eine Strömungsleitstruktur, insbesondere einen Strömungsleitkörper, zur wenigstens teilweisen laminaren Ausrichtung des Fluidstroms umfasst. Eine derartige Strömungsleitstruktur ist ein in die Gesamtvorrichtung integriertes Bauteil, das eine gezielte und besonders effiziente laminare Ausrichtung des Fluidstroms ermöglicht und somit vorteilhaft innerhalb des Baukammereintrittsbereichs angeordnet werden kann. Mittels der Strömungsleitstruktur lässt sich der Fluidstrom derart leiten dass die gewünschte laminare Anströmung im Bereich der Baukammer erzielt werden kann. Der Strömungsleitkörper kann beispielsweise einen Diffusor umfassen. Dabei ist denkbar, dass der Diffusor beispielsweise als dünnwandiger Hohlkegel ausgebildet ist, dessen Mantelfläche so geformt ist, dass sich der Hohlkegel in Richtung auf die Baukammer erweitert.

Weiterhin kann die Strömungsausrichtungseinheit so ausgelegt sein, dass sich um das Bauteil ein Luftvorhang bildet und damit das Bauteil nicht direkt angeströmt wird. Hierdurch wird es möglich, den Bauteilverzug zu reduzieren. Dies wird insbesondere dadurch ermöglicht, weil kein direkter Luftstrom auf das Bauteil trifft, wodurch eine gleichmäßige Abkühlung des Bauteils ermöglicht wird.

Des Weiteren kann vorgesehen sein, dass das Fluidversorgungssystem wenigstens eine Fluidsterilisations- und/oder Fluidfiltervorrichtung aufweist, welche mit der Fluiddruckerzeugungsvorrichtung, der Fluiderhitzungsvorrichtung und dem Baukammer-Gehäuse in Fluidverbindung steht. Aufgrund der Fluidsterilisations- und/oder Fluidfiltervorrichtung kann das der Baukammer zugeführte Fluid besonders effizient gefiltert und/oder gereinigt werden. Die Fluidsterilisations- und/oder Fluidfiltervorrichtung ist dabei stromaufwärts (bezogen auf die Durchströmungsrichtung der Baukammer) angeordnet. Durch geeignete Ausgestaltung dieser Vorrichtung kann das Fluid vor Eintritt in die Baukammer derart effizient gereinigt, sterilisiert und gefiltert werden, dass die Baukammer den Vorschriften bzw. Normen der EN ISO 14644 für Reinräume entspricht. Besonders vorteilhaft ist in diesem Zusammenhang der geschlossene Fluidkreislauf zu nennen. Denn durch die kontinuierliche Umwälzung des Fluids innerhalb des Fluidversorgungssystems wird ab der ersten Umwälzung das bereits gereinigte bzw. gefilterte Fluid erneut der Fluidsterilisations- und/oder Fluidfiltervorrichtung zugeführt. Demzufolge kann die Filterung bzw. Sterilisation des Fluids mit jedem Umlauf des Fluids vorgenommen werden, was sich entscheidend auf die Sterilität und die Partikelbelastung des herzustellenden Bauteils auswirkt.

Ebenfalls ist vorstellbar, dass der Fluidstrom eine Temperatur in einem Bereich insbesondere von ca. 20°C bis ca. 400 °C, vorzugsweise von ca. 30°C bis ca. 350 °C und besonders bevorzugt von ca. 50°C bis ca. 300 °C aufweist. Die zuvor beschriebenen Temperaturbereiche, insbesondere der Temperaturbereich von ca. 50°C bis ca. 300 °C, sind besonders vorteilhaft für eine möglichst verzugsfreie Herstellung von Thermoplasten mittels des 3D-Druckers. Thermoplaste können beispielsweise aus der Gruppe der Hochleistungsthermoplaste ausgewählt werden.

Die Hochleistungsthermoplaste können beispielsweise Polyetherketone (wie PAEK, PEKK, PEEK, PEEEK, PEEKK, PEKEKK), Polyamide (wie PA 69, PA 612, PA 11, PA 12, PA 46, PA 1212, PA 6/12, PA 1010, PPA), Polyphenylensulfid PPS, Polyamidimide, Polysulfone (wie PAS, PSF, PES, PPSU, PSU, PESU) und/oder Polycarbonate PC und auch thermoplastische Polyimide (PEI, PAI, PESI) sein. Zudem ist denkbar, dass die Thermoplaste faser- und/oder partikelverstärkt sind.

Darüber hinaus ist denkbar, dass der Fluidstrom innerhalb der Baukammer eine, insbesondere mittlere, Geschwindigkeit in einem Bereich insbesondere von ca. 0,05 m/s bis ca. 5 m/s, vorzugsweise von ca. 0,1 m/s bis ca. 5 m/s und besonders bevorzugt von ca. 0,2 m/s bis ca. 3 m/s aufweist. Durch die zuvor genannten Bereiche der mittleren Geschwindigkeit, insbesondere der Geschwindigkeitsbereich von ca. 0,2 m/s bis ca. 3 m/s, des Fluidstroms kann eine sichere, insbesondere konstante und homogene Beheizung der Baukammer des 3D-Druckers gewährleistet werden. Diese Art der Beheizung hilft vor allem dabei, den Bauteilverzug bei der Verarbeitung von Thermoplasten deutlich zu reduzieren. Im Übrigen ist zur Erfüllung der vorstehend genannten Norm eine Durchströmung der Baukammer durch ein Fluid vorgeschrieben.

Weiterhin ist möglich, dass die Fluidsterilisations- und/oder Fluidfiltervorrichtung, die Fluiddruckerzeugungsvorrichtung, die Fluiderhitzungsvorrichtung und/oder das Baukammer-Gehäuse bis zu einer Maximaltemperatur von ca. 300 °C temperaturbeständig sind. Bis zu einer Maximaltemperatur von ca. 300 °C des Fluids können im Wesentlichen alle relevanten technischen Thermoplaste durch den 3D-Drucker verarbeitet werden. Eine Maximaltemperatur von ca. 300 °C ist daher insbesondere für die verzugsfreie Verarbeitung von Thermoplasten besonders effizient, vorteilhaft und auch notwendig.

Zusätzlich kann vorgesehen sein, dass die Fluiddruckerzeugungsvorrichtung eine Strömungsmaschine, wie z.B. ein Turboverdichter, insbesondere ein Radialverdichter bzw. ein Ventilator bzw. Radialventilator, ist. Grundsätzlich ist unter einer Strömungsmaschine beispielsweise ein Ventilator oder ein Verdichter zu verstehen. Diese Art der Verdichter erzeugt ein besonders puls- bzw. stoßarmen und damit konstanten druckbehafteten Fluidstrom, so dass sich die Druckschwankungen und Temperaturschwankungen innerhalb der Baukammer ebenfalls minimieren lassen können. Demzufolge kann eine noch homogenere Temperaturverteilung innerhalb der Baukammer ausgebildet werden. Der Radialverdichter kann insbesondere auch als ein bis zu ca. 300 °C temperaturbeständiger Radialventilator ausgebildet sein.

Ferner ist vorstellbar, dass stromabwärts des Turboverdichters wenigstens eine Druckverringerungsvorrichtung angeordnet ist. Die Druckverringerungsvorrichtung ist deshalb besonders vorteilhaft einsetzbar, weil durch sie ein bestimmter Gegendruck für den Turboverdichter erzeugt wird, der die Verwendung eines bis zu 300 °C temperaturbeständigen Radialventilators bzw. Radialgebläses physikalisch zwingend erforderlich ist. Die Druckverringerungsvorrichtung kann beispielsweise als Lochblech oder als Lochblende ausgestaltet sein.

Auch denkbar ist, dass durch die Druckverringerungsvorrichtung, die Fluidsterilisations- und/oder Fluidfiltervorrichtung, die Fluiderhitzungsvorrichtung und/oder die Strömungsausrichtungseinheit wenigstens eine Druckverringerung des Fluidstroms von wenigstens 50 Pa erzeugbar ist. Diese durch die zuvor benannten Bauteile hervorgerufene Druckverringerung wirkt sich besonders vorteilhaft auf den Betrieb bzw. die Steuer- und/oder Regelbarkeit des Turboverdichters, insbesondere des temperaturbeständigen Radialverdichters, aus. Ferner kann ein in seinen Abmaßen für den 3D-Drucker geeigneter Radialverdichter besonders effizient und vorteilhaft bei einem derartigen Druckverlust betrieben werden.

Überdies ist möglich, dass das Fluidversorgungssystem eine Partikelmessvorrichtung zur Überwachung des Betriebs des 3D-Druckers aufweist, die im Baukammereintrittsbereich zwischen der Strömungsausrichtungseinheit und wenigstens einer Eintrittsöffnung des Baukammer-Gehäuses angeordnet ist. Eine solche Partikelmessvorrichtung ist für die Erzielung eines hohen, vor allem in der Medizintechnik geforderten Bauteilstandards hinsichtlich Sterilität und Partikelbelastung besonders wichtig und vorteilhaft. Durch den Einsatz dieser Partikelmessvorrichtung kann zudem die Überwachung des gesamten Fluidversorgungssystems des 3D-Druckers optimiert werden, da ausgehend von der Partikelmessung beispielsweise durch eine zentrale elektronische Steuerungs- und oder Regelungseinheit des 3D-Druckers eine Wartung bzw. ein Austausch der Fluidsterilisations- und/oder Fluidfiltervorrichtung vorgeschlagen werden kann. Ferner kann auf Basis der Partikelmessung auch ein 3D-Druckvorgang abgebrochen werden, wenn ein gewisser Grenzwert für Partikel innerhalb des Fluidstroms überschritten wird. Ebenfalls denkbar ist, dass auf der Grundlage der Partikelmessung die Fluidsterilisations- und/oder Fluidfiltervorrichtung mittels eines Bypassventils und einer Bypassleitung überbrückt werden können. Das Bypassventil kann die Bypassleitung dabei so lange freigeben, bis ein einstellbarer Grenzwert für Partikel innerhalb des Fluidstroms überschritten wird. Insbesondere beim Einsatz von sehr teuren Fluidsterilisations- und/oder Fluidfiltervorrichtungen kann die Bypassleitung zu einer spürbaren Verlängerung ihrer Lebensdauer und zu verlängerten Wartungsintervallen führen.

Weiter kann vorgesehen sein, dass der Fluidstrom ein Fluid enthält, das ein Gas, insbesondere Luft, ist. Der Einsatz eines Gases, insbesondere Luft, ist vor allem aufgrund der nahezu unbegrenzten Verfügbarkeit von Luft und der dadurch sehr einfachen Handhabbarkeit besonders vorteilhaft.

Zudem vorstellbar ist, dass das Fluidversorgungssystem wenigstens einen Gasanschluss aufweist, mittels dessen das Fluidversorgungssystem mit wenigstens einem anderen Prozessgas als Luft befüllbar ist, wobei der 3D-Drucker während der Befüllung nicht in Betrieb ist. In diesem Zusammenhang ist es denkbar, dass das Prozessgas ein Inertgas ist. Durch den Einsatz des Inertgas wird insbesondere der Oxidation bei der Verarbeitung von oxidationsanfälligen Thermoplasten durch den 3D-Drucker vorgebeugt, wodurch die Bauteilqualität nochmals verbessert werden kann.

Weitere Einzelheiten und Vorteile der Erfindung sollen nun anhand eines in den Zeichnungen dargestellten Ausführungsbeispiels näher erläutert werden.

Es zeigen:
- Fig. 1: eine schematische Darstellung eines Ausführungsbeispiels eines erfindungsgemäßen Fluidversorgungssystems für einen 3D-Drucker;
- Fig. 2: eine Teilschnittdarstellung eines Baukammereintrittsbereichs des Fluidversorgungssystems gemäß Fig. 1 und eines darin angeordneten Strömungsleitkörpers;
- Fig. 3: eine schematische, perspektivische Darstellung eines Aufnahmegestells für den 3D-Drucker und für das Fluidversorgungssystem gemäß Fig. 1; und
- Fig. 4: eine schematische Vorderansicht der Baukammer des 3D-Druckers gemäß Fig. 1.

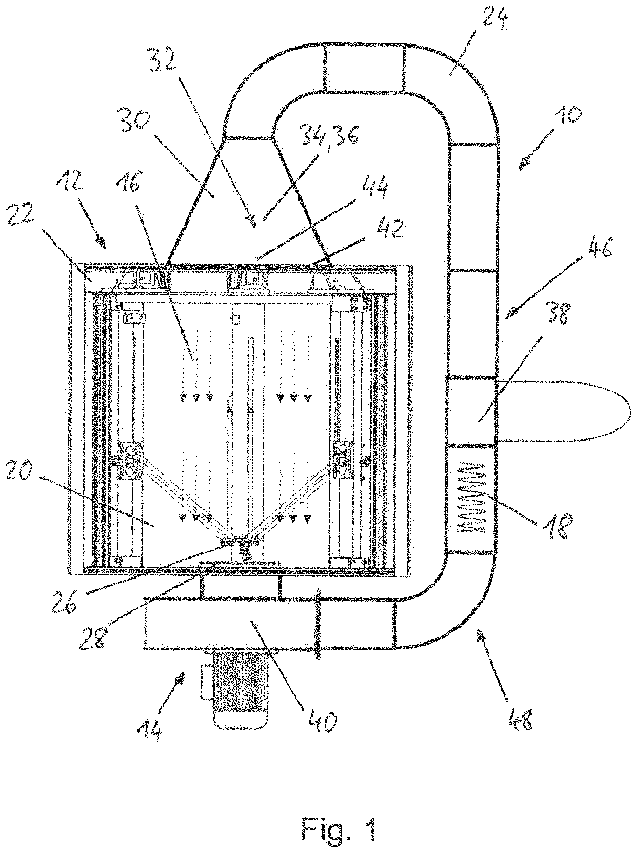

Fig. 1 zeigt eine schematische Darstellung eines Ausführungsbeispiels eines erfindungsgemäßen Fluidversorgungssystems 10 für einen 3D-Drucker 12.

Als 3D-Drucker 12 kann beispielsweise ein 3D-Drucker 12 mit einer Delta-Kinematik oder auch mit einem kartesischen System verwendet werden. Grundsätzlich kann es sich bei dem 3D-Drucker 12 auch um einen mehrdimensionalen Drucker und/oder einen Drucker mit einem mehrachsigen Drucksystem handeln.

Der 3D-Drucker 12 ist als ein FFF-3D-Drucker (FFF: engl. für Fused Filament Fabrication) ausgebildet.

Das Fluidversorgungssystem 10 umfasst eine Fluiddruckerzeugungsvorrichtung 14 zur Erzeugung eines druckbeaufschlagten Fluidstroms 16.

Der Fluidstrom 16 enthält ein Fluid, das ein Gas ist.

Das Gas kann entweder Luft oder ein Prozessgas wie ein Inertgas sein.

Des Weiteren umfasst Fluidversorgungssystem 10 einer Fluiderhitzungsvorrichtung 18 zur Erhitzung des Fluidstroms 16.

Der 3D-Drucker 12 weist weiter eine Baukammer 20 auf, die mittels eines Baukammer-Gehäuses 22 gegenüber der Umgebung des 3D-Druckers 12 begrenzt und fluiddicht abgeschlossen ist.

Außerdem stehen die Fluiddruckerzeugungsvorrichtung 14, die Fluiderhitzungsvorrichtung 18 und das Baukammer-Gehäuse 22 in Fluidverbindung. Somit ist die Baukammer 20 mittels des Fluidstroms 16 durchströmbar.

Der Fluidstrom 16 weist innerhalb der Baukammer 20 eine mittlere Geschwindigkeit in einem Bereich von ca. 0,2 m/s bis ca. 3 m/s auf.

Der Fluidstrom 16 kann außerdem innerhalb der Baukammer 20 eine mittlere Geschwindigkeit in einem Bereich von ca. 0,05 m/s bis ca. 5 m/s aufweisen.

Zudem ist denkbar, dass der Fluidstrom 16 innerhalb der Baukammer 20 eine mittlere Geschwindigkeit in einem Bereich von ca. 0,1 m/s bis ca. 5 m/s aufweisen kann.

Die Fluiddruckerzeugungsvorrichtung 14, der Fluiderhitzungsvorrichtung 18 und das Baukammer-Gehäuse 22 bilden im Übrigen einen geschlossenen Fluidkreislauf 24 für den Fluidstrom 16 aus.

Der Fluidstrom 16 weist insbesondere eine Temperatur in einem Bereich von ca. 50°C bis ca. 300 °C auf.

Jedoch ist es ebenfalls vorstellbar, dass der Fluidstrom 16 eine Temperatur in einem Bereich von ca. 20°C bis ca. 400 °C aufweisen kann.

Zudem ist es denkbar, dass der Fluidstrom 16 eine Temperatur in einem Bereich von ca. 30°C bis ca. 350 °C aufweisen kann.

Der Fluidstrom 16 weist diese Temperaturbereiche insbesondere innerhalb der Baukammer 20 auf.

Der Fluidstrom 16 wird zudem bereits vor Eintritt in die Baukammer 20 mittels der Fluiderhitzungsvorrichtung 18 erhitzt.

Der 3D-Drucker 12 weist weiterhin einen innerhalb der Baukammer 20 mehrachsig bewegbaren Druckkopf 26 und eine Bauplattform 28 auf.

Der Druckkopf 26 und die Bauplattform 28 sind von der laminaren Strömung des Fluidstroms 16 im betriebsbereiten Zustand des 3D-Druckers 12 umgeben.

Das Fluidversorgungssystem 10 weist ferner einen Baukammereintrittsbereich 30 auf.

Der Baukammereintrittsbereich 30 ist stromaufwärts des Baukammer-Gehäuses 22 angeordnet.

Innerhalb des Baukammereintrittsbereichs 30 ist eine Strömungsausrichtungseinheit 32 angeordnet.

Die Strömungsausrichtungseinheit 32 umfasst eine Strömungsleitstruktur 34.

Die Strömungsleitstruktur ist als Strömungsleitkörper 36 ausgebildet.

Das Fluidversorgungssystem 10 weist gemäß Fig. 1 weiter eine Fluidsterilisations- und Fluidfiltervorrichtung 38 auf.

Die Fluidsterilisations- und Fluidfiltervorrichtung 38 steht mit der Fluiddruckerzeugungsvorrichtung 14, der Fluiderhitzungsvorrichtung 18 und dem Baukammer-Gehäuse 22 in Fluidverbindung.

Die Fluiddruckerzeugungsvorrichtung 14 ist im Übrigen ein Radialverdichter 40.

Stromabwärts des Radialverdichters 40 (bezogen auf die Flussrichtung des Fluids durch die Vorrichtung) ist eine Druckverringerungsvorrichtung 42 angeordnet.

Das Fluidversorgungssystem 10 weist weiter eine Partikelmessvorrichtung 44 zur Überwachung des Betriebs des 3D-Druckers 12 auf.

Die Partikelmessvorrichtung 44 ist im Baukammereintrittsbereich 30 zwischen der Strömungsausrichtungseinheit 32 und einer Eintrittsöffnung des Baukammer-Gehäuses 22 angeordnet.

Darüber hinaus weist das Fluidversorgungssystem 10 einen Gasanschluss 46 auf.

Das in Fig. 1 dargestellte Fluidversorgungssystem 10 umfasst zudem ein Rohrsystem 48, das den geschlossenen Fluidkreislauf 24 ausbildet.

Das Rohrsystem 48 besteht mehreren geraden Rohrstücken und 90°-Krümmern, durch welche der Radialverdichter 40, die Fluiderhitzungsvorrichtung 18, die Fluidsterilisations- und Fluidfiltervorrichtung 38, der Baukammereintrittsbereich 30 sowie die Baukammer 20 miteinander in Fluidverbindung stehen.

Der Radialverdichter 40 ist an einem Baukammeraustritt an dem Baukammer-Gehäuse 22 angeflanscht, wohingegen der Baukammereintrittsbereich 30 an einem Baukammereintritt an dem Baukammer-Gehäuse 22 angeflanscht ist.

Zwischen dem Radialverdichter 40 und dem Baukammereintrittsbereich 30 sind die Fluiderhitzungsvorrichtung 18 und die Fluidsterilisations- und Fluidfiltervorrichtung 38 in dem Rohrsystem 48 angeordnet.

Die Fiuiderhitzungsvorrichtung 18 ist dabei stromabwärts des Radialverdichters 40 angeordnet.

Die Fluidsterilisations- und Fluidfiltervorrichtung 38 ist wiederum stromabwärts der Fiuiderhitzungsvorrichtung 18 in dem Rohrsystem 48 angeordnet.

Die Fluiderhitzungsvorrichtung 18 ist als Durchlauferhitzer ausgebildet, der ein elektrisches Heizelement aufweist, das z.B. in Form einer Heizspirale ausgebildet sein kann.

Fluidsterilisations- und Fluidfiltervorrichtung 38 kann beispielsweise eine EPA-, HEPA- oder ULPA-Filtereinheit aufweisen.

Die Fluidsterilisations- und Fluidfiltervorrichtung 38 kann zudem einen Abscheidegrad gemäß den Filterklassen: E10, E11, E12, H13, H14, U15, U16 oder U17 aufweisen.

Die Funktion des Fluidversorgungssystems 10 lässt sich nun wie folgt beschreiben: Vor Inbetriebnahme des 3D Druckers 12 sollte zunächst durch das Fluidversorgungssystem 10 überprüft werden, ob das für den Druckvorgang und den Druckwerkstoff geeignete Gas in der Baukammer 20 enthalten ist.

Diese Überprüfung kann beispielsweise mit einem Gassensor erfolgen, der innerhalb des Rohrsystems 48 oder in der Baukammer 20 positioniert ist, und in der Lage ist das entsprechende Gas zu bestimmen.

Die Bestimmung unterschiedlicher Gase kann auch den Einsatz von mehreren Gassensoren nötig machen.

Die Befüllung des Fluidversorgungssystems 10 mit Luft als Prozessgas (nachdem das zuvor verwendete Gas evakuiert wurde) kann beispielsweise über ein Be- und Entlüftungsventil erfolgen, das in dem Rohrsystem 48 angeordnet ist.

Zur Unterstützung bzw. Beschleunigung des Befüllungsvorgangs mit Luft kann der Radialverdichter 40 zur Hilfe genommen werden.

Während der Befüllung ist der 3D-Drucker 12 nicht in Betrieb.

Nach erfolgter Befüllung des Fluidversorgungssystems 10 wird das Fluidversorgungssystem 10 durch Schließen des Be- und Entlüftungsventils (nicht in Fig. 1 gezeigt) von der Umgebungsatmosphäre luftdicht abgeschlossen.

Noch vor dem Betrieb des 3D-Druckers 12 kann die Luft innerhalb des Fluidversorgungssystems 10 mittels des Radialverdichters 40 umgewälzt werden, wodurch eine Vorreinigung der Luft erreicht wird.

Während dieser Vorreinigung kann die Fluiderhitzungsvorrichtung 18 bereits in Betrieb sein, wodurch es zusätzlich zu einer Vorerhitzung der Baukammer 20 kommt. Sobald die Baukammer 20 eine an den Werkstoff und das herzustellende Bauteil angepasste Baukammertemperatur aufweist, nimmt der 3D-Drucker 12 seinen Betrieb auf.

Während dem Betrieb des 3D-Druckers 12 wird die die Baukammer 20 verlassende Luft von dem Radialverdichter 40 angesaugt, verdichtet und der Fluiderhitzungsvorrichtung 18 zugeführt.

Dort wird die Luft auf einen einstellbaren bzw. regelbaren Temperaturwert erhitzt und nach Ausströmen aus der Fluiderhitzungsvorrichtung 18 der Fluidsterilisations- und Fluidfiltervorrichtung 38 zugeführt, wo sie gereinigt und gefiltert wird und anschließend stromabwärts in den Baukammereintrittsbereich 30 einströmt.

Die Fluidsterilisations- und Fluidfiltervorrichtung 38, die Fluiddruckerzeugungsvorrichtung 14 in Form des Radialverdichters 40, die Fluiderhitzungsvorrichtung 18 und das Baukammer-Gehäuse 22 sind im Übrigen bis zu einer Maximaltemperatur von ca. 300 °C temperaturbeständig. Übliche Anwendungsfälle können aber auch geringere Maximaltemperaturen im Bereich zwischen 150-200°C erfordern.

Während der Durchströmung des Baukammereintrittsbereichs 30, dessen Gehäuse als Diffusor ausgebildet ist und in dem der Strömungsleitkörper 36 angeordnet ist, kommt es zur laminaren Ausrichtung des Fluidstroms 16.

Der Strömungsleitkörper 36 dient somit zumindest teilweise zur laminaren Ausrichtung des Fluidstroms 16.

Jedoch dient zusätzlich auch der Diffusor zur laminaren Ausrichtung des Fluidstroms 16.

Das Fluidversorgungssystem 10 ist demnach im betriebsbereiten Zustand so ausgestaltet, dass die Baukammer 20 mittels des Fluidstroms 16 in Form einer laminaren Strömung durchströmbar ist.

Innerhalb des Baukammereintrittsbereichs 30 kann die Partikelmessvorrichtung 44 zudem vor dem Eintritt der Luft in die Baukammer 20 die Partikelanzahl messen und als Messgröße einer elektronischen Regelungseinheit zur Verfügung stellen.

Die Regelungseinheit (nicht in Fig. 1 gezeigt) dient zur Regelung eines Antriebs (z.B. ein Elektromotor) des Radialverdichters 40 sowie der Fluiderhitzungsvorrichtung 18.

Die Regelungseinheit kann in das Fluidversorgungssystem 10 integriert sein oder an dem Baukammer-Gehäuse 22 angeordnet sein.

Zudem ist die Regelungseinheit mit sämtlichen Sensoren elektrisch verbunden, die in dem Fluidversorgungssystem 10 und in dem Baukammer-Gehäuse 20 angeordnet sind.

Das Fluidversorgungssystem 10 und der 3D-Drucker 12 können demnach einen oder mehrere Druck- und Temperatursensoren aufweisen.

Zwischen dem Baukammereintrittsbereich 30 und dem Baukammer-Gehäuse 22 kann ferner die Druckverringerungsvorrichtung 42 (z.B. in Form eines Lochblechs) angeordnet sein, welche den für den Betrieb des Radialverdichters 40 notwendigen Gegendruck zumindest teilweise erzeugt.

Der Gegendruck ist notwendig, um ein ungebremstes "Hochdrehen" des Radialverdichters 40, also eine ungebremste Drehzahlerhöhung des Radialverdichters 40 zu verhindern.

Neben der Druckverringerungsvorrichtung 42 können weitere Elemente teilnehmen an der Funktion der Druckverringerung, wie beispielsweise das bzw. ein Leitwerk (z.B. der Strömungsleitkörper 36) und auch die Fluidsterilisations- und Fluidfiltervorrichtung 38.

Nach erfolgter Durchströmung der Druckverringerungsvorrichtung 42 strömt der Fluid- bzw. Luftstrom 16 in die Baukammer 20 ein.

Durch die Druckverringerungsvorrichtung 42, die Fluidsterilisations- und Fluidfiltervorrichtung 38, die Fluiderhitzungsvorrichtung 18 und die Strömungsausrichtungseinheit 32 ist somit eine Druckverringerung des Fluidstroms von wenigstens 50 Pa erzeugbar.

Die Baukammer 20 wird sodann von dem bereits erhitzten Fluid- bzw. Luftstrom 16 in Form einer laminaren Strömung durchströmt, so dass dieser zu der gewünschten Erhitzung der Baukammer 20 und zu einer möglichst verzugsfreien Herstellung eines Bauteils mittels des 3D-Druckers 12 beiträgt.

Zudem sollte der durch den Fluidstrom 16 innerhalb der Baukammer 20 erzeugte Druck stets höher als der die Baukammer 20 umgebende Druck sein, um eine zusätzliche Partikelbelastung und Keimbelastung aus der Umgebung bzw. Atmosphäre zu vermeiden.

Nach Durchströmung der Baukammer 20 strömt die Luft aus dieser aus und wird erneut von dem Radialverdichter 40 angesaugt, so dass der zuvor beschriebene Vorgang wiederholt wird.

Dieser Vorgang wird so lange wiederholt, bis der Druckvorgang abgeschlossen ist oder beispielsweise eine Störung auftritt (z.B. eine zu hohe Partikelkonzentration).

Der zuvor beschriebene Vorgang kann auch mit jedem anderen Prozessgas als Luft wie zuvor beschrieben ablaufen, so dass Luft als Fluid nur beispielshaft zu betrachten ist.

Soll der zuvor beschriebene Vorgang mit einem anderen Prozessgas (z.B. Inertgas) als Luft ablaufen, muss die Luft aus dem Fluidversorgungssystem 10 sowie der Baukammer 20 des 3D-Druckers evakuiert werden.

Mit Hilfe des Gasanschlusses 46 ist sodann das Fluidversorgungssystem 10 mit einem anderen Prozessgas als Luft befüllbar.

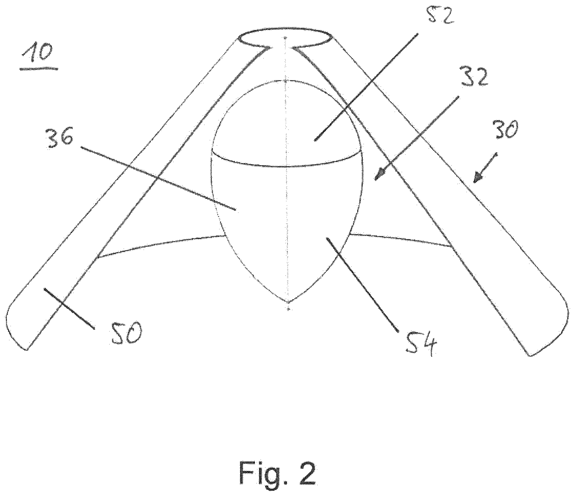

Fig. 2 zeigt eine Teilschnittdarstellung eines Baukammereintrittsbereichs 30 des Fluidversorgungssystems 10 gemäß Fig. 1 und eines darin angeordneten Strömungsleitkörpers 36.

Der Baukammereintrittsbereich 30 besteht aus einem Diffusor 50 sowie der an dem Diffusor 50 befestigten Strömungsausrichtungseinheit 32 in Form des Strömungsleitkörpers 36.

Denn der Diffusor 50 ist als Hohlkegel ausgebildet, dass dessen Mantelfläche so geformt ist, dass diese sich trichterförmig in Richtung der Baukammer (z.B. linear) erweitert.

Zur Befestigung des Strömungsleitkörpers 36 an der Innenwand des Diffusors 50 können zwei oder mehr strömungsoptimierte Befestigungsstreben (nicht in Fig. 2 gezeigt) vorgesehen sein.

Der Strömungsleitkörper 36 gemäß Fig. 2 ist tropfenförmig ausgebildet und zwar durch eine Halbkugel 52 und einem an der größten Querschnittsfläche der Halbkugel bündig anschließenden in Richtung auf die Baukammer spitz zulaufenden Rotationskörper 54.

Der Strömungsleitkörper 54 verjüngt sich ausgehend von der Halbkugel 52 entlang der Längsachse bzw. Rotationsache des Strömungsleitkörpers 36 und mündet in eine Spitze.

Der Strömungsleitkörper 36 kann aus den zuvor beschriebenen Körpern zusammengesetzt sein oder einstückig ausgebildet sein.

Der Rotationskörper 36 und der Diffusor 50 sind koaxial zueinander ausgerichtet.

Die Spitze des Strömungsleitkörpers 36 ist im Übrigen der Baukammer 20 zugewandt.

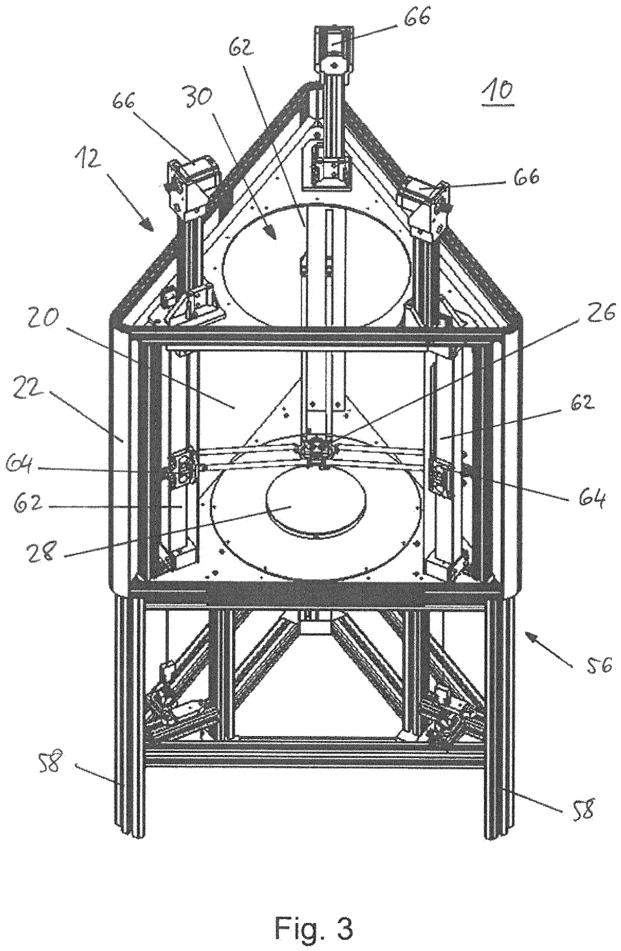

Fig. 3 zeigt eine schematische, perspektivische Darstellung eines Aufnahmegestells 56 für den 3D-Drucker 12 sowie für das Fluidversorgungssystem 10 gemäß Fig. 1.

Das Aufnahmegestell 56 besteht aus drei vertikalen Profilstützen 58, die entsprechend den Ecken eines gleichseitigen Dreiecks zueinander ausgerichtet sind und fachwerkartig durch zusätzliche Querstreben verstrebt sind. Grundsätzlich ist es jedoch nicht erforderlich, dass der Grundriss dreieckig ist. Denkbar sind auch quadratische, rechteckige oder sonstige Grundformen bzw. Grundrisse.

An dem Aufnahmegestell 56 ist das Baukammer-Gehäuse 22 befestigt, das einen äußeren fachwerkartigen Rahmen 60 aufweist, dessen Querschnitt ebenfalls die Form eines gleichseitigen Dreiecks aufweist.

Der Rahmen 60 weist im montierten Zustand eine obere und eine untere Platte auf, wobei die untere Platte in Schwerkraftrichtung unterhalb der oberen Platte angeordnet ist, so dass die obere Platte mit einer gewissen Höhe zu der unteren Platte beabstandet ist.

Die obere und untere Platte sind ebenfalls beide als gleichseitiges Dreieck ausgebildet, wodurch die Baukammer 20 zusätzlich zu dem äußeren Rahmen 60 begrenzt wird.

Die obere Platte ist zudem mit dem Baukammereintrittsbereich 30 gekoppelt, wohingegen die untere Platte die Bauplattform trägt und mit einem Gehäuse des Radialverdichters 40 gekoppelt ist.

Die drei Eckbereiche der oberen und unteren Platte sind jeweils durch eine vertikal ausgerichtete Linearführung 62 (also insgesamt drei Linearführungen 62) miteinander verbunden, deren Länge der Höhe zwischen oberer und unterer Platte entspricht.

An der Außenkontur jeder Linearführung 62 ist weiter ein dazu vertikal verschieblicher Schlitten 64 geführt.

Jeder der drei Schlitten 64 weißt zwei Kugelgelenke auf, an welchen jeweils ein stabförmiger Arm mit seinem ersten Ende angelenkt ist.

Mit seinem zweiten Ende ist jeder Arm zudem an dem Druckkopf 26 angelenkt, der dafür ebenfalls zwei Kugelgelenke pro Armpaar aufweist.

Insgesamt weist der Druckkopf 26 gemäß Fig. 3 also drei Kugelgelenkpaare auf, die jeweils hin zu einem Schlitten 64 ausgerichtet sind und an denen jeweils zwei Armpaare ausgehend von den drei Schlitten 64 angelenkt sind.

Im montierten Zustand den Druckkopfes 26 ist zudem jedes Armpaar parallel zueinander ausgerichtet.

Die dreidimensionale Bewegung des Druckkopfes 26 innerhalb der Baukammer 20 folgt somit aus dessen kinematischer Anlenkung an die drei Schlitten 64 mittels der Armpaare sowie aus den jeweiligen vertikalen Linearbewegungen der drei Schlitten 64.

Die vertikale Linearbewegung eines Schlittens 64 erfolgt über einen elektrischen Schrittmotor 66, der mit einer Stütze an der oberen Platte oberhalb dieser befestigt ist. Anstelle eines elektrischen Schrittmotors 66 kann auch grundsätzlich ein Servomotor oder eine andere Antriebseinheit eingesetzt werden.

Ausgehend von dem Schrittmotor 66 erstreckt sich ein Riemen (z.B. ein Zahnriemen) innerhalb der gesamten Länge der Linearführung 62, die hohl ausgebildet ist.

An dem Riemen ist ein magnetisches Koppelelement (z.B. ein weiterer innengeführter Schlitten) befestigt, welches die Linearbewegung des Riemens mittels magnetischem Schluss an den außengeführten Schlitten 64 überträgt, der an der Außenkontur der Linearführung 62 angeordnet ist. Alternativ kann vorgesehen sein, dass der Schlitten auch direkt angetrieben wird, beispielsweise durch ein Seilzugsystem oder einen Gewindetrieb. Hierdurch kann auf einen Innenschlitten und auf eine magnetische Kopplung verzichtet werden.

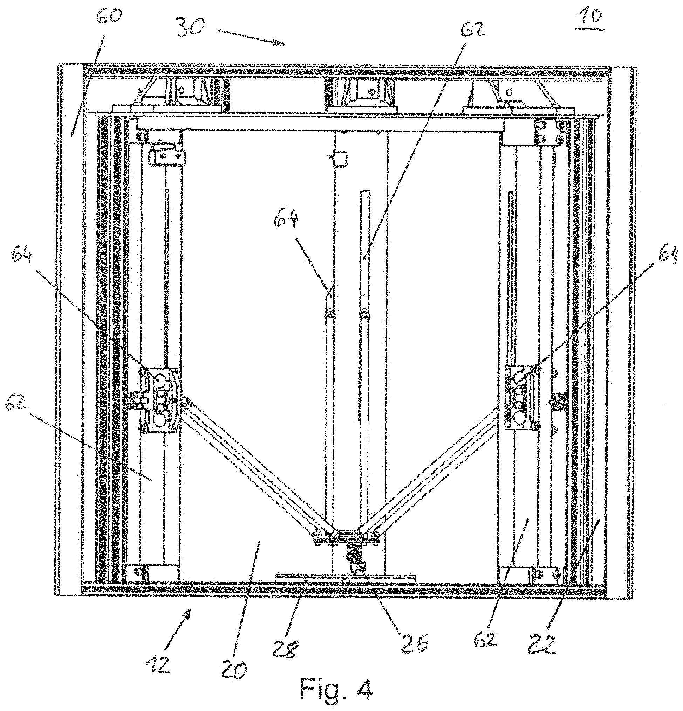

Fig. 4 zeigt eine noch detailliertere schematische Vorderansicht der Baukammer 20 des 3D-Druckers 12 gemäß Fig. 1 und 3.

### Bezugszeichenliste

- 10: Fluidversorgungssystem
- 12: FFF-3D-Drucker
- 14: Fluiddruckerzeugungsvorrichtung
- 16: Fluidstrom
- 18: Fluiderhitzungsvorrichtung, Durchlauferhitzer
- 20: Baukammer
- 22: Baukammer-Gehäuse
- 24: Fluidkreislauf
- 26: Druckkopf
- 28: Bauplattform
- 30: Baukammereintrittsbereich
- 32: Strömungsausrichtungseinheit
- 34: Strömungsleitstruktur
- 36: Strömungsleitkörper
- 38: Fluidsterilisations- und Fluidfiltervorrichtung
- 40: Radialverdichter
- 42: Druckverringerungsvorrichtung
- 44: Partikelmessvorrichtung
- 46: Gasanschluss
- 48: Rohrsystem
- 50: Diffusor
- 52: Halbkugel
- 54: Rotationskörper
- 56: Aufnahmegestell
- 58: Profilstützen
- 60: Rahmen
- 62: Linearführung
- 64: Schlitten
- 66: Schrittmotor

## Patentansprüche

1. 3D-Drucker (12), insbesondere ein FFF-3D-Drucker (12), mit einem Fluidversorgungssystem (10), das wenigstens eine Fluiddruckerzeugungsvorrichtung (14) zur Erzeugung eines druckbeaufschlagten Fluidstroms (16) und wenigstens eine Fluiderhitzungsvorrichtung (18) zur Erhitzung des Fluidstroms (16) aufweist, wobei der 3D-Drucker (12) wenigstens eine Baukammer (20) aufweist, die mittels wenigstens eines Baukammer-Gehäuses (22) gegenüber der Umgebung des 3D-Druckers (12) begrenzt und fluiddicht abgeschlossen ist, wobei die Fluiddruckerzeugungsvorrichtung (14), die Fluiderhitzungsvorrichtung (18) und das Baukammer-Gehäuse (22) in Fluidverbindung stehen, wodurch die Baukammer (20) mittels des Fluidstroms (16) durchströmbar ist, und wobei mittels der Fluiddruckerzeugungsvorrichtung (14), der Fluiderhitzungsvorrichtung (18) und des Baukammer-Gehäuses (22) ein geschlossener Fluidkreislauf (24) für den Fluidstrom (16) ausgebildet ist, der vor Eintritt in die Baukammer (20) mittels der Fluiderhitzungsvorrichtung (18) erhitzt ist, wobei das Fluidversorgungssystem (10) im betriebsbereiten Zustand so ausgestaltet ist, dass die Baukammer (20) mittels des Fluidstroms (16) in Form einer im Wesentlichen laminaren Strömung durchströmbar ist, und
wobei das Fluidversorgungssystem (10) wenigstens einen Baukammereintrittsbereich (30) aufweist, der stromaufwärts des Baukammer-Gehäuses (22) angeordnet ist und in dem wenigstens eine Strömungsausrichtungseinheit (32) angeordnet ist, wobei der Baukammereintrittsbereich (30) in Form eines Diffusors ausgebildet ist.

2. 3D-Drucker (12) nach Anspruch 1, **dadurch gekennzeichnet, dass** der 3D-Drucker (12) wenigstens einen innerhalb der Baukammer (20) mehrachsig und/oder mehrdimensional, insbesondere dreidimensional, bewegbaren Druckkopf (26) und wenigstens eine Bauplattform (28) aufweist, die von der laminaren Strömung des Fluidstroms (16) umgeben sind.

3. 3D-Drucker (12) nach Anspruch 1 oder Anspruch 2, **dadurch gekennzeichnet, dass** die Strömungsausrichtungseinheit (32) eine Strömungsleitstruktur (34), insbesondere einen Strömungsleitkörper (36), zur wenigstens teilweisen laminaren Ausrichtung des Fluidstroms (16) umfasst.

4. 3D-Drucker (12) nach einem der vorhergehenden Ansprüche, **dadurch gekennzeichnet, dass** das Fluidversorgungssystem (10) wenigstens eine Fluidsterilisations- und/oder Fluidfiltervorrichtung (38) aufweist, welche mit der Fluiddruckerzeugungsvorrichtung (14), der Fluiderhitzungsvorrichtung (18) und dem Baukammer-Gehäuse (22) in Fluidverbindung steht.

5. 3D-Drucker (12) nach Anspruch 1, **dadurch gekennzeichnet, dass** der Fluidstrom (16) eine Temperatur in einem Bereich insbesondere von ca. 20°C bis ca. 400 °C, vorzugsweise von ca. 30°C bis ca. 350 °C und besonders bevorzugt von ca. 50°C bis ca. 300 °C aufweist.

6. 3D-Drucker (12) nach Anspruch 1, **dadurch gekennzeichnet, dass** der Fluidstrom (16) innerhalb der Baukammer (20) eine, insbesondere mittlere, Geschwindigkeit in einem Bereich insbesondere von ca. 0,05 m/s bis ca. 5 m/s, vorzugsweise von ca. 0,1 m/s bis ca. 5 m/s und besonders bevorzugt von ca. 0,2 m/s bis ca. 3 m/s aufweist.

7. 3D-Drucker (12) nach Anspruch 3, **dadurch gekennzeichnet, dass** die Fluidsterilisations- und/oder Fluidfiltervorrichtung (38), die Fluiddruckerzeugungsvorrichtung (14), die Fluiderhitzungsvorrichtung (18) und/oder das Baukammer-Gehäuse (22) bis zu einer Maximaltemperatur von ca. 300 °C temperaturbeständig sind.

8. 3D-Drucker (12) nach Anspruch 1, **dadurch gekennzeichnet, dass** die Fluiddruckerzeugungsvorrichtung (14) eine Strömungsmaschine beispielsweise ein Turboverdichter (40), insbesondere ein Radialverdichter (40) oder ein Ventilator, ist.

9. 3D-Drucker (12) nach Anspruch 8, **dadurch gekennzeichnet, dass** stromabwärts der Strömungsmaschine (40) wenigstens eine Druckverringerungsvorrichtung (42) angeordnet ist.

10. 3D-Drucker (12) nach Anspruch 9, **dadurch gekennzeichnet, dass** durch die Druckverringerungsvorrichtung (42), die Fluidsterilisations- und/oder Fluidfiltervorrichtung (38), die Fluiderhitzungsvorrichtung (18) und/oder die Strömungsausrichtungseinheit (32) wenigstens eine Druckverringerung des Fluidstroms von wenigstens 50 Pa erzeugbar ist.

11. 3D-Drucker (12) nach einem der vorhergehenden Ansprüche, **dadurch gekennzeichnet, dass** das Fluidversorgungssystem (10) eine Partikelmessvorrichtung (44) zur Überwachung des Betriebs des 3D-Druckers (12) aufweist, die im Baukammereintrittsbereich (30) zwischen der Strömungsausrichtungseinheit (32) und wenigstens einer Eintrittsöffnung des Baukammer-Gehäuses (22) angeordnet ist.

12. 3D-Drucker (12) nach einem der vorhergehenden Ansprüche, **dadurch gekennzeichnet, dass** der Fluidstrom (16) ein Fluid enthält, das ein Gas, insbesondere Luft, ist.

13. 3D-Drucker (12) nach einem der vorhergehenden Ansprüche, **dadurch gekennzeichnet, dass** das Fluidversorgungssystem (10) wenigstens einen Gasanschluss (46) aufweist, mittels dessen das Fluidversorgungssystem (10) mit wenigstens einem anderen Prozessgas als Luft befüllbar ist, wobei der 3D-Drucker (12) während der Befüllung nicht in Betrieb ist.

## Claims

1. A 3D printer (12), in particular an FFF 3D printer (12), having a fluid supply system (10) which comprises at least one fluid pressure generating device (14) for generating a pressurized fluid stream (16) and at least one fluid heating device (18) for heating the fluid stream (16), the 3D printer (12) comprising at least one building chamber (20) which is delimited from the environment of the 3D printer (12) by means of at least one building chamber housing (22) and is sealed in a fluid-tight manner, the fluid pressure generating device (14), the fluid heating device (18) and the building chamber housing (22) being in fluid communication, as a result of which the fluid stream (16) can flow through the building chamber (20), and a closed fluid circuit (24) for the fluid stream (16) being formed by means of the fluid pressure generating device (14), the fluid heating device (18) and the building chamber housing (22), which fluid stream (16) is heated by means of the fluid heating device (18) before entering the building chamber (20), the fluid supply system (10) in the operational state being designed such that the fluid stream (16) can flow through the building chamber (20) in the form of a substantially laminar flow, and
the fluid supply system (10) comprising at least one building chamber inlet area (30), which is arranged upstream of the building chamber housing (22) and in which at least one flow alignment unit (32) is arranged, the building chamber inlet area (30) being implemented in the form of a diffusor.

2. The 3D printer (12) according to claim 1, **characterized in that** the 3D printer (12) comprises at least one print head (26), which can be moved within the building chamber (20) in several axes and/or several dimensions, in particular in three dimensions, and at least one building platform (28), which are surrounded by the laminar flow of the fluid stream (16).

3. The 3D printer (12) according to claim 1 or claim 2, **characterized in that** the flow alignment unit (32) comprises a flow guide structure (34), in particular a flow guide body (36), for an at least partial laminar alignment of the fluid stream (16).

4. The 3D printer (12) according to any of the preceding claims, **characterized in that** the fluid supply system (10) comprises at least one fluid sterilization and/or fluid filtering device (38) in fluid communication with the fluid pressure generating device (14), the fluid heating device (18) and the building chamber housing (22).

5. The 3D printer (12) according to claim 1, **characterized in that** the fluid stream (16) has a temperature in a range in particular from about 20 °C to about 400 °C, preferably from about 30 °C to about 350 °C and particularly preferred from about 50 °C to about 300 °C.

6. The 3D printer (12) according to claim 1, **characterized in that** the fluid stream (16) within the building chamber (20) has a velocity, in particular an average velocity, in a range in particular from about 0.05 m/s to about 5 m/s, preferably from about 0.1 m/s to about 5 m/s and particularly preferred from about 0.2 m/s to about 3 m/s.

7. The 3D printer (12) according to claim 3, **characterized in that** the fluid sterilization and/or fluid filtering device (38), the fluid pressure generating device (14), the fluid heating device (18) and/or the building chamber housing (22) are temperature-resistant up to a maximum temperature of about 300 °C.

8. The 3D printer (12) according to claim 1, **characterized in that** the fluid pressure generating device (14) is a turbomachine, for example a turbo compressor (40), in particular a centrifugal compressor (40) or a fan.

9. The 3D printer (12) according to claim 8, **characterized in that** at least one pressure reduction device (42) is arranged downstream of the turbomachine (40).

10. The 3D printer (12) according to claim 9, **characterized in that** at least a pressure reduction of the fluid stream of at least 50 Pa can be generated by the pressure reduction device (42), the fluid sterilization and/or fluid filtering device (38), the fluid heating device (18) and/or the flow alignment unit (32).

11. The 3D printer (12) according to any of the preceding claims, **characterized in that** the fluid supply system (10) comprises a particle measuring device (44) for monitoring the operation of the 3D printer (12), which is arranged in the building chamber inlet area (30) between the flow alignment unit (32) and at least one inlet opening of the building chamber housing (22).

12. The 3D printer (12) according to any of the preceding claims, **characterized in that** the fluid stream (16) contains a fluid that is a gas, in particular air.

13. The 3D printer (12) according to any of the preceding claims, **characterized in that** the fluid supply system (10) comprises at least one gas connection (46) by means of which the fluid supply system (10) can be filled with at least one process gas other than air, the 3D printer (12) not being in operation during the filling process.

## Revendications

1. Imprimante 3D (12), notamment imprimante 3D FFF (12), avec un système d'alimentation en fluide (10) qui comporte au moins un dispositif de production de pression fluide (14) pour produire un flux de fluide chargé en pression (16) et au moins un dispositif de chauffage de fluide (18) pour chauffer le flux de fluide (16), l'imprimante 3D (12) comportant au moins une chambre modulaire (20) délimitée à l'aide d'au moins un carter de chambre modulaire (22) par rapport à l'environnement de l'imprimante 3D (12) et etanchéifiée de façon étanche aux fluides, le dispositif de production de pression fluide (14), le dispositif de chauffage de fluide (18) et le carter de chambre modulaire (22) étant en liaison fluide, faisant que la chambre modulaire (20) peut être traversée par le flux de fluide (16) et un circuit de fluide fermé (24) étant réalisé pour le flux de fluide (16) à l'aide du dispositif de production de pression fluide (14), du dispositif de chauffage de fluide (18) et du carter de chambre modulaire (22), ledit circuit étang chauffé avant l'entrée dans la chambre modulaire (20) à l'aide du dispositif de chauffage de fluide (18), le système d'alimentation en fluide (10) étant configuré de telle sorte dans l'état prêt à fonctionner que la chambre modulaire (20) peut être traversée à l'aide du flux de fluide (16) sous la forme d'un flux pour l'essentiel laminaire ; et
le système d'alimentation en fluide (10) comportant au moins une zone d'entrée de chambre modulaire (30) disposée vers l'amont du carter de chambre modulaire (22) et étant réalisée dans l'au moins une unité d'orientation de flux (32), la zone d'entrée de chambre modulaire (30) étant réalisée sous la forme d'un diffuseur.

2. Imprimante 3D (12) selon la revendication 1, **caractérisée en ce que** l'imprimante 3D (12) comporte au moins une tête d'impression (26) mobile de façon pluriaxiale et/ou pluridimensionnelle, notamment de façon tridimensionnelle, à l'intérieur de la chambre modulaire (20) ainsi qu'au moins une plateforme modulaire (28), ces éléments étant ceints par le flux laminaire du flux de fluide (16).

3. Imprimante 3D (12) selon la revendication 1 ou 2, **caractérisée en ce que** l'unité d'orientation de flux (32) comprend une structure de conduction de flux (34), notamment un corps de conduction de flux (36), pour l'orientation laminaire au moins partielle du flux de fluide (16).

4. Imprimante 3D (12) selon l'une quelconque des revendications précédentes, **caractérisée en ce que** le système d'alimentation en fluide (10) comporte au moins un dispositif de filtrage de fluide et/ou de stérilisation de fluide (38) qui est en liaison fluide avec le dispositif de production de pression fluide (14), le dispositif de chauffage de fluide (18) et le carter de chambre modulaire (22) .

5. Imprimante 3D (12) selon la revendication 1, **caractérisée en ce que** le flux de fluide (16) présente une température située dans une plage notamment d'environ 20°C à environ 400 °C, de préférence d'environ 30°C à environ 350 °C et de façon particulièrement préférée d'environ 50°C à environ 300 °C.

6. Imprimante 3D (12) selon la revendication 1, **caractérisée en ce que** le flux de fluide (16) présent à l'intérieur de la chambre modulaire (20) présente une vitesse, notamment médiane, située dans une plage notamment d'environ 0,05 m/s à environ 5 m/s, de préférence d'environ 0,1 m/s à environ 5 m/s et de façon particulièrement préférée d'environ 0,2 m/s à environ 3 m/s.

7. Imprimante 3D (12) selon la revendication 3, **caractérisée en ce que** le dispositif de filtrage de fluide et/ou de stérilisation de fluide (38), le dispositif de production de pression fluide (14), le dispositif de chauffage de fluide (18) et/ou le carter de chambre modulaire (22) sont résistants à la température jusqu'à une température maximale d'environ 300 °C.

8. Imprimante 3D (12) selon la revendication 1, **caractérisée en ce que** le dispositif de production de pression fluide (14) est une machine d'écoulement par exemple un turbocompresseur (40), notamment un compresseur radial (40) ou un ventilateur.

9. Imprimante 3D (12) selon la revendication 8, **caractérisée en ce qu'**au moins un dispositif de réduction de pression (42) est disposé en aval de la machine d'écoulement (40).

10. Imprimante 3D (12) selon la revendication 9, **caractérisée en ce qu'**au moins une réduction de pression d'au moins 50 Pa du flux de fluide peut être réalisée par le biais du dispositif de réduction de pression (42), du dispositif de filtrage de fluide et/ou de stérilisation de fluide (38), du dispositif de chauffage de fluide (18) et/ou de l'unité d'orientation de flux (32).

11. Imprimante 3D (12) selon l'une quelconque des revendications précédentes, **caractérisée en ce que** le système d'alimentation en fluide (10) comporte un dispositif de mesure de particules (44) pour surveiller le fonctionnement de l'imprimante 3D (12), ledit dispositif étant disposé dans la zone d'entrée de chambre modulaire (30) entre l'unité d'orientation de flux (32) et au moins une ouverture d'entrée du carter de chambre modulaire (22).

12. Imprimante 3D (12) selon l'une quelconque des revendications précédentes, **caractérisée en ce que** le flux de fluide (16) contient un fluide qui est un gaz, notamment de l'air.

13. Imprimante 3D (12) selon l'une quelconque des revendications précédentes, **caractérisée en ce que** le système d'alimentation en fluide (10) comporte au moins un raccord de gaz (46) à l'aide duquel le système d'alimentation en fluide (10) peut être rempli d'au moins un autre gaz de traitement que l'air, l'imprimante 3D (12) ne pouvant pas fonctionner pendant le remplissage.
